# FASCICULE DE BREVET EUROPEEN

(11) **EP 3 687 379 B1**
(45) Date de publication et mention de la délivrance du brevet: **06.11.2024**
(21) Numéro de dépôt: 18778920.1
(22) Date de dépôt: 28.09.2018
(51) Int. Cl.: A61B 5/00, A61B 5/11

(54) **DISPOSITIF ET METHODE POUR DETECTER QU'UNE PERSONNE ALITEE QUITTE SON LIT OU A CHUTE**
VORRICHTUNG UND VERFAHREN ZUR ERKENNUNG, OB EINE BETTLÄGERIGE PERSON IHR BETT VERLÄSST ODER GEFALLEN IST
DEVICE AND METHOD FOR DETECTING IF A BEDRIDDEN PERSON LEAVES HIS OR HER BED OR HAS FALLEN

(30) Priorité: 29.09.2017 BE 201705693
(43) Date de publication de la demande: 05.08.2020
(73) Titulaire: Kapcare SA, 1170 Bruxelles (BE)
(72) Inventeur: KAPLAN, Philippe, 1160 Bruxelles (BE)
(74) Mandataire: Icosa Europe
(86) Numéro de dépôt international: PCT/EP2018/076501
(87) Numéro de publication internationale: WO 2019/063808

(56) Documents cités:
- EP-A1- 2 589 330
- WO-A1-2015/114902
- JP-A- 2003 290 154
- US-A1- 2012 140 068
- US-A1- 2017 116 484

## Description

### Domaine de l'invention

L'invention se rapporte au domaine des dispositifs de surveillance de personnes alitées, en particulier les dispositifs et méthodes permettant de déterminer si une personne alitée quitte son lit, soit volontairement, soit involontairement tel que dans le cas d'une chute du lit par exemple, ou si une personne a chuté quelque part en dehors de son lit.

### État de la technique

La détection du fait qu'une personne quitte volontairement son lit ou qu'elle en est tombée sont des préoccupations importantes, notamment dans les centres hospitaliers et les maisons de retraite. Cette détection est par exemple importante lorsque la personne est sujette à des conditions ou des circonstances particulières, telles qu'une maladie (notamment la démence, Alzheimer,...), une situation post-opératoire, ou un historique de chutes. Cette surveillance est particulièrement recherchée dans les unités de soin, telles que des hôpitaux, des cliniques de soin ou des maisons de retraite, afin de prévenir toute détérioration de la condition de patients, bien que cette surveillance puisse également se faire dans d'autres milieux.

Le rythme au sein des unités de soin empêche qu'un personnel qualifié puisse surveiller l'ensemble des personnes alitées pour détecter ces événements. Ainsi, différents dispositifs ont été développés pour les détecter. A titre d'exemples, des dispositifs détecteurs de pression peuvent être disposés sous le matelas du lit, voire sur les appuis au sol du lit. Lorsque la pression mesurée ne correspond pas à la pression attendue d'une personne alitée, une alarme peut être déclenchée. Ces détecteurs manquent cependant de finesse, notamment lorsqu'il s'agit de prévenir le levé du patient. Ainsi, un patient qui tente de se lever ne déclenche pas l'alerte, ce qui peut engendrer des complications, par exemple si le patient chute en tentant de quitter son lit. Ce dispositif ne permet donc pas d'anticiper une quelconque chute ou un levé indésirable, et ne permet que de constater que la personne alitée n'est plus dans son lit. Ces dispositifs sont aussi dépendants du poids de la personne et manquent de fiabilité pour des personnes de faible poids, ce qui est souvent le cas pour une personne très âgée. Ils posent aussi la question de l'entretien, de la mise en place et requièrent donc du temps de manipulation de la part du personnel.

Des dispositifs alternatifs ont donc été mis au point. A titre d'exemple, on peut citer le brevet européen EP 2335232 qui décrit un système d'alerte de sortie du lit et une méthode de prédiction de la vraisemblance qu'un utilisateur sorte d'un lit. Ce système comprend un ou plusieurs capteurs aptes à mesurer une caractéristique physiologique pour produire des signaux indicatifs de la caractéristique physiologique, et un processeur qui surveille ces signaux et détermine, en fonction des changements des signaux, si une alarme doit être déclenchée car l'utilisateur est vraisemblablement en train de quitter le lit. Cependant, la méthode décrite et le dispositif associé manque de finesse dans le traitement des signaux, et peut générer des fausses alertes.

### Résumé de l'invention

Un but de l'invention est de fournir un dispositif et une méthode permettant de déterminer avec une meilleure fiabilité si un individu tente de quitter son lit et/ou quitte son lit et/ou est tombé. Par une meilleure fiabilité, on entend un dispositif et/ou une méthode générant moins de faux positifs et/ou de faux négatifs que les méthodes et/ou dispositifs connus. Un autre but de l'invention est de fournir un dispositif qui soit facile à installer et à utiliser.

L'invention est définie par les revendications indépendantes. Les revendications dépendantes définissent des modes de réalisation préférés de l'invention.

L'invention fournit un dispositif pour détecter si une personne alitée quitte son lit ou a quitté son lit ou a chuté après avoir quitté son lit, le dispositif comprenant un détecteur ayant un champ de détection apte à couvrir au moins une partie du lit et au moins une partie de son environnement, ledit détecteur comportant plusieurs capteurs de distance, chaque capteur de distance étant apte à fournir des mesures de distance au cours du temps entre ledit capteur et un obstacle correspondant dans ledit champ de détection, ledit dispositif comprenant en outre une unité de traitement reliée au détecteur et configurée pour traiter les mesures de distance fournies par les capteurs de distance du détecteur. Le dispositif est caractérisé en ce que l'unité de traitement est configurée pour déterminer la position dans l'espace de chacun desdits obstacles au cours du temps et en fonction de la distance mesurée par le détecteur pour l'obstacle en question, et pour classer chaque obstacle selon que l'obstacle est situé dans un premier volume ou dans un deuxième volume dans l'espace, le premier volume étant un volume s'étendant verticalement vers le haut et/ou vers le bas à partir de la surface dudit lit, le deuxième volume étant un volume s'étendant vers l'extérieur à partir des limites latérales du premier volume, et en ce que l'unité de traitement est configurée pour déterminer un événement concernant ladite personne si la distance mesurée pour au moins un obstacle situé dans le deuxième volume varie de plus qu'une première valeur prédéterminée.

La mesure d'une variation de distance associée à un obstacle situé dans le deuxième volume permet de fournir à l'unité de traitement une information relative à la présence d'un mouvement au sein du deuxième volume, qui est un volume bordant le lit. Ce mouvement est caractéristique du fait que la personne alitée quitte son lit.

Le dispositif selon l'invention est plus fiable que celui faisant appel à des capteurs de pression puisqu'il ne se préoccupe pas de ce qui se passe dans le lit. En particulier, il n'est pas influencé par les caractéristiques physiologiques de la personne, telles que son poids par exemple. Il est aussi plus facile à installer et à utiliser puisqu'il ne doit pas être relié au lit.

Le dispositif selon l'invention est aussi plus fiable et plus facile à utiliser que celui décrit dans le brevet européen EP 2335232 car ce dernier fait également appel à la mesure de caractéristiques physiologiques de la personne alitée, alors ce ces caractéristiques peuvent varier fort d'une personne à l'autre.

De préférence, l'unité de traitement est configurée pour classer chaque obstacle selon que l'obstacle est situé dans le premier volume ou dans le deuxième volume ou dans un troisième volume, le troisième volume étant un volume s'étendant vers l'extérieur à partir des limites latérales extérieures du deuxième volume, et l'unité de traitement est configurée pour déterminer un événement concernant ladite personne si la distance mesurée pour au moins un obstacle situé dans le deuxième volume varie de plus qu'une première valeur prédéterminée, à condition qu'aucune des distances mesurées pour les obstacles situé dans le troisième volume n'aient varié de plus qu'une deuxième valeur prédéterminée pendant un premier laps de temps précédent le moment où la distance mesurée pour l'au moins un obstacle situé dans le deuxième volume a varié de plus que la première valeur prédéterminée.

Ceci permet d'éviter que le fait qu'une tierce personne entre dans la pièce où se trouve le lit et se déplace jusqu'au bord du lit, ne soit interprétée par le dispositif comme le fait que la personne alitée quitte son lit.

De préférence, l'unité de traitement est configurée pour déterminer un événement concernant ladite personne si les distances mesurées pour au moins un nombre prédéterminé d'obstacles situés dans le deuxième volume varient chacune de plus qu'une première valeur prédéterminée, ledit nombre prédéterminé d'obstacles étant supérieur ou égal à deux.

Ceci permet d'éviter qu'un événement soit détecté s'il n'y a pas assez de volume en mouvement. Un tel dispositif préféré ne détectera donc rien dans le cas où par exemple uniquement la main du patient dépasse du lit.

De préférence, l'unité de traitement est configurée pour calculer la position du centre géométrique d'un groupe d'obstacles situés dans le deuxième volume et dont les distances mesurées varient chacune de plus que la première valeur prédéterminée, et pour déterminer que la personne est tombée du lit dans lequel elle était alitée lorsque ledit centre géométrique se situe à une hauteur inférieure à une hauteur de référence. Ceci permet en effet de détecter une chute de lit de manière plus fiable et efficace et par exemple de ne pas considérer comme une chute, le fait que la personne alitée s'assied simplement sur le bord de son lit. Cela permet aussi de détecter qu'un personne chute après être sortie normalement de son lit et avoir par exemple fait quelques pas dans le deuxième volume.

La présente invention concerne également une méthode pour détecter si une personne alitée quitte son lit, la méthode comprenant les étapes suivantes :
a) mise en place d'un détecteur de manière à ce qu'un champ de détection du détecteur couvre au moins une partie du lit et au moins une partie de son environnement, ledit détecteur comportant plusieurs capteurs de distance, chaque capteur de distance étant apte à fournir des mesures de distance au cours du temps entre ledit capteur et un obstacle correspondant dans ledit champ de détection, ledit détecteur étant relié à une unité de traitement configurée pour traiter les mesures de distance fournies par les capteurs de distance du détecteur,
b) calibration du détecteur et de l'unité de traitement pour définir un espace à surveiller dans le champ de détection du détecteur, cet espace à surveiller étant divisé en au moins un premier volume et un deuxième volume, le premier volume étant un volume s'étendant verticalement vers le haut et/ou vers le bas à partir de la surface dudit lit, le deuxième volume étant un volume s'étendant vers l'extérieur à partir des limites latérales du premier volume,
c) déterminer, au moyen de l'unité de traitement, la position dans l'espace de chacun desdits obstacles au cours du temps et en fonction de la distance mesurée par le détecteur pour l'obstacle en question,
d) classer, au moyen de l'unité de traitement, chaque obstacle selon que l'obstacle est situé dans un premier volume ou dans un deuxième volume dans l'espace,
e) déterminer, au moyen de l'unité de traitement, qu'un événement concernant ladite personne a lieu si la distance mesurée pour au moins un obstacle situé dans le deuxième volume varie de plus qu'une première valeur prédéterminée.

### Brève description des figures

Ces aspects ainsi que d'autres aspects de l'invention seront clarifiés dans la description détaillée de modes de réalisation particuliers de l'invention, référence étant faite aux dessins des figures, dans lesquelles :
- Fig.1: montre schématiquement une vue d'ensemble d'un dispositif selon l'invention ;
- Fig.2: montre une vue du haut du dispositif de la Fig.1 selon un mode de réalisation;
- Fig.3: montre une vue du haut du dispositif de la Fig.1 selon un mode de réalisation préféré de l'invention ;

Les dessins des figures ne sont ni à l'échelle, ni proportionnés. Généralement, des capteurs semblables ou identiques sont dénotés par des références identiques dans les figures.

### Description détaillée de modes de réalisation de l'invention

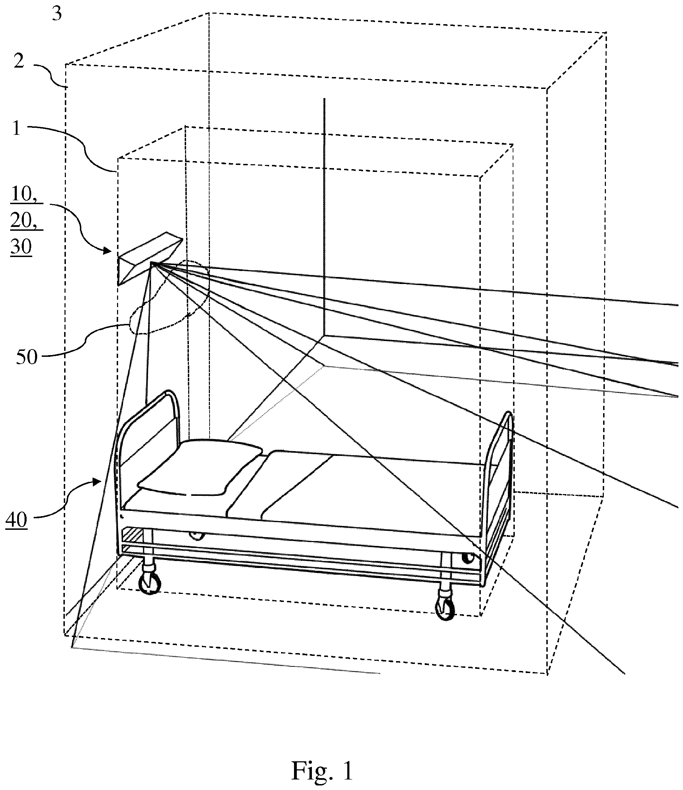

La Fig.1 montre schématiquement une vue d'ensemble d'un dispositif (10) selon l'invention lorsqu'il est monté par exemple dans une chambre dans laquelle est installée un lit (40) sur lequel est alité une personne (la personne non représentée) dont il faut surveiller si elle quitte ledit lit ou si elle a chuté.

Le dispositif (10) comprend un détecteur (20) ayant un champ de détection (50) apte à couvrir au moins une partie du lit (40) et au moins une partie de son environnement. Le champ de détection est bien entendu cette partie de l'espace dans laquelle le détecteur est apte à remplir sa fonction. Ledit détecteur (20) comporte plusieurs capteurs de distance, chaque capteur de distance étant apte à fournir des mesures de distance au cours du temps entre ledit capteur et un obstacle correspondant dans ledit champ de détection (50). Le détecteur (20) peut par exemple être une caméra fonctionnant sur le principe du temps de vol (anglais : Time of Flight, TOF) et qui permet de mesurer directement ou indirectement et en temps réel des distances par rapport à une scène en trois dimensions observée. Pour ce faire, une caméra TOF illumine la scène qui se trouve dans son champ de détection et calcule le temps que la lumière émise prend pour effectuer le trajet entre chaque élément photosensible (parfois aussi appelé « pixel » dans ce contexte) de la caméra et un obstacle correspondant dans son champ de vision.

Etant donné que la vitesse de la lumière est constante, ce temps de vol est directement proportionnel à la distance entre ledit élément photosensible et l'obstacle correspondant. Cette mesure de temps de vol est effectuée indépendamment pour chaque élément photosensible (ou « pixel ») de la caméra. Dans l'exemple d'une caméra TOF, il faut donc comprendre par « un capteur de distance étant apte à fournir des mesures de distance au cours du temps entre ledit capteur et un obstacle correspondant », l'ensemble d'un élément photosensible de la caméra et les moyens dont dispose la caméra pour calculer le temps de vol pour ledit élément photosensible.

Un exemple concret d'un tel détecteur est la caméra « SwissRanger 4000 » ou « SR4000 » de MESA Imaging, qui comprend une matrice de 176 × 144 éléments photosensibles.

Le détecteur (20) est préférentiellement placé ou configuré pour être placé à une hauteur par rapport au sol qui est supérieure à la hauteur maximale de la surface supérieure dudit lit et il est orienté ou conçu pour être orienté pour que son champ de détection (50) couvre au moins une partie du lit (40), de préférence la totalité du lit, et au moins une partie de son environnement.

A titre d'exemple, comme illustré à la Fig. 1, le détecteur (20) peut être placé contre un mur au-dessus de la tête de lit (40), mais d'autres positionnements sont envisageables, comme par exemple contre un mur opposé à celui contre lequel le lit est placé. Le détecteur (20) peut également être disposé de façon à avoir dans son champ de détection (50), d'une part au moins une partie du lit, de préférence sa totalité, et d'autre part une porte permettant de sortir et d'entrer dans la pièce où est disposé le détecteur (20).

Le dispositif (10) comprend en outre une unité de traitement (30) reliée au détecteur (20) et configurée pour acquérir et traiter les mesures de distance (ou de temps de vol, ce qui est équivalent à un facteur constant près) fournies par les capteurs de distance du détecteur au cours du temps. L'unité de traitement est de préférence apte à mémoriser les différentes mesures fournies par les capteurs de distance.

En particulier, l'unité de traitement (30) est configurée pour déterminer la position dans l'espace de chacun desdits obstacles au cours du temps et en fonction de la distance mesurée par le détecteur pour l'obstacle en question. La détermination de ces positions se fait par un calcul géométrique bien connu, par exemple dans l'état de l'art des caméras TOF et de leurs applications. L'unité de traitement (30) est également configurée pour - une fois ces positions connues - classer chaque obstacle selon que l'obstacle est situé dans un premier volume (1) ou dans un deuxième volume (2) dans l'espace. Le premier volume (1) est un volume s'étendant verticalement vers le haut et/ou vers le bas à partir de la surface dudit lit et est donc typiquement le volume où la personne se trouve lorsqu'elle est alitée. Le deuxième volume (2) est un volume s'étendant vers l'extérieur à partir des limites latérales du premier volume.

Le premier et le deuxième volume n'ont donc de préférence pas de points communs, mis à part éventuellement leur frontière commune. Le deuxième volume peut être de dimensions finies ou infinies. Le deuxième volume peut avoir ou ne pas avoir de frontières extérieures.

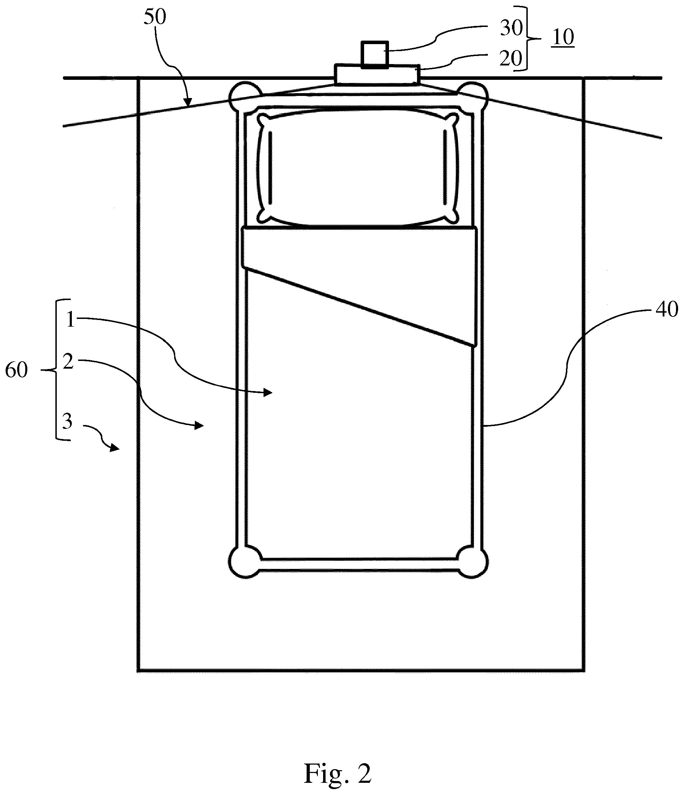

Le Fig.1 montre un exemple de premier volume (1) et de deuxième volume (2), en lignes pointillées. Bien que cela n'apparaisse pas clairement sur la figure, il faut comprendre que ces deux volumes sont de préférence exclusifs, c'est-à-dire qu'ils n'ont pas de points communs, mis à part éventuellement leur frontière commune qui, dans le cas de la Fig.1, est l'enveloppe extérieure du premier volume (1). La Fig.2 montre une vue du haut du dispositif de la Fig.1 selon un mode de réalisation, ce qui permet de voir les projections orthogonales du premier volume et du deuxième volume sur le sol.

Dans cet exemple, les capteurs de distance qui pointent par exemple vers l'oreiller du lit fourniront chacun, lorsque le lit n'est pas occupé, la distance entre le capteur et une zone correspondante sur l'oreiller dans sa ligne de visée. Les valeurs de ces distances permettront à l'unité de traitement (30) de déterminer que les points correspondants de l'oreiller se trouvent dans le premier volume (1), alors que les capteurs de distance qui pointent par exemple vers les parties du sol qui sont à proximité de l'empreinte au sol du lit fourniront chacun la distance entre le capteur et une zone correspondante au sol dans sa ligne de visée. La valeur de ces distances qui permettront à l'unité de traitement de déterminer que les points correspondants au sol se trouvent dans le deuxième volume (2).

L'unité de traitement (30) est par ailleurs configurée pour déterminer un événement concernant ladite personne si la distance mesurée pour au moins un obstacle situé dans le deuxième volume varie de plus qu'une première valeur prédéterminée (un premier seuil). En d'autres termes, l'unité de traitement déterminera qu'un événement concernant la personne a lieu si l'unité de traitement détecte qu'un mouvement significatif a lieu dans le deuxième volume (2). L'événement en question peut être le fait que la personne tente de sortir ou sorte de son lit, ou qu'elle soit tombée de son lit, ou qu'elle soit tombée quelque part en dehors de son lit.

L'unité de traitement (30) peut évaluer ladite variation de distance de manière périodique ou continue, dans le dernier cas dans les limites de la cadence maximale à laquelle le détecteur peut fournir les informations de distance. Dans le cas d'une évaluation périodique, l'unité de traitement peut évaluer ladite variation de distance par exemple toutes les 5 secondes, toutes les 4 secondes, toutes les 3 secondes, toutes les 2 secondes, toutes les secondes, ou toutes les 0.5 seconde. Alternativement, un état initial enregistré en un temps *t0* peut servir de point de comparaison pour les variations de distance mesurées à chaque instant *t* suivant *to.* Le temps entre le temps *t0* et l'instant *t* suivant pouvant alors être supérieur à 1 minute, ou à 5 minutes, ou à 10 minutes. Il s'entend que l'état initial à *t0* peut être réinitialisé de façon régulière, par exemple toutes les 10 minutes, ou toutes les 30 minutes, voire toutes les heures.

L'unité de traitement (30) peut être tout moyen permettant de recevoir et d'analyser les mesures de distances fournies par le détecteur (20), et ce en fonction du temps. A titre d'exemple, il peut s'agir d'un microprocesseur comprenant un programme d'analyse des données correspondantes aux mesures distances fournies par le détecteur.

Notons que l'unité de traitement (30) peut être regroupée avec le détecteur (20), par exemple au sein d'un même boitier. Alternativement, le détecteur (20) peut être séparé de l'unité de traitement (30) et être doté de moyens de communication de données, de préférence sans-fil, tel que par exemple par Wl-FI (norme IEEE 802.11), permettant le transfert de données vers l'unité de traitement (30). L'unité de traitement (30) comprend alors des moyens de réception des données transmises par le détecteur (20) via les moyens de communication. Lesdites données comprennent dans ce cas au moins les mesures distances ou les temps de vol fournis par les capteurs de distance du détecteur (20).

De préférence, l'unité de traitement (30) est configurée pour classer chaque obstacle selon que l'obstacle est situé dans le premier volume (1) ou dans le deuxième volume (2) ou dans un troisième volume (3). Le troisième volume étant dans ce cas un volume s'étendant vers l'extérieur à partir des limites latérales extérieures du deuxième volume, ce qui implique, dans ce cas préféré, que le deuxième volume possède des limites extérieures finies et bien définies. Dans l'exemple illustré à la Fig.1, le troisième volume (3) est le volume de l'espace qui entoure le deuxième volume (2) sans le contenir. Le deuxième et le troisième volume n'ont donc de préférence pas de points communs, mis à part éventuellement leur frontière commune.

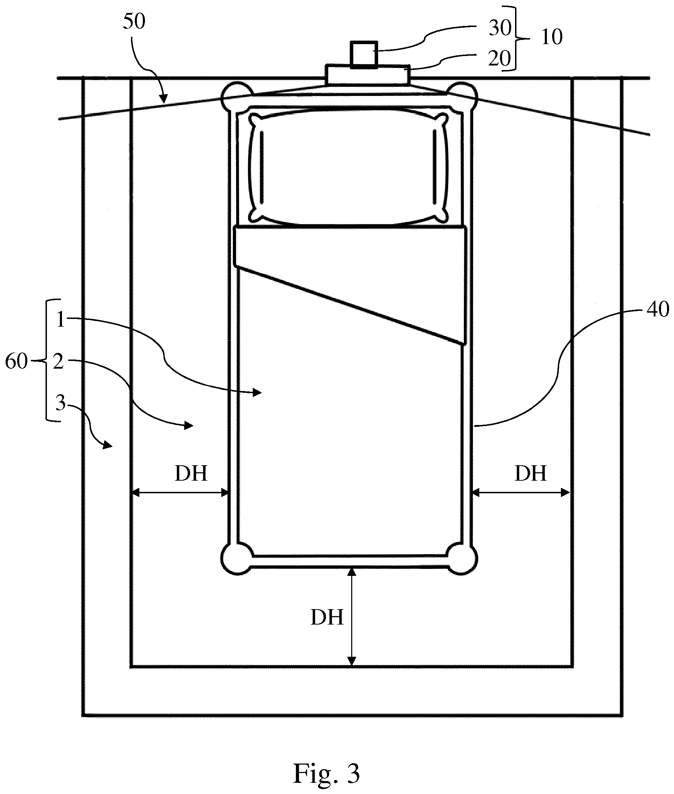

La Fig.3 montre une vue du haut du dispositif de la Fig.1 selon ce mode de réalisation préféré de l'invention, ce qui permet de voir les projections orthogonales du premier volume (1), du deuxième volume (2), et du troisième volume (3) sur le sol. Dans le cas de la Fig.3, le troisième volume possède des frontières extérieures. Il est à noter que le troisième volume peut ne pas avoir de frontières extérieures et s'étendre à l'infini (cas non illustré).

Dans cette version préférée, l'unité de traitement (30) est configurée pour déterminer un événement concernant ladite personne si la distance mesurée pour au moins un obstacle situé dans le deuxième volume (2) varie de plus qu'une première valeur prédéterminée, à condition qu'aucune des distances mesurées pour les obstacles situés dans le troisième volume (3) n'aient varié de plus qu'une deuxième valeur prédéterminée pendant un premier laps de temps précédent le moment où la distance mesurée pour l'au moins un obstacle situé dans le deuxième volume (2) a varié de plus que la première valeur prédéterminée.

En d'autres termes, l'unité de traitement déterminera qu'un événement concernant la personne a lieu si l'unité de traitement détecte qu'un mouvement significatif a lieu dans le deuxième volume sans que ce mouvement ne soit précédé au cours du premier laps de temps d'un autre mouvement significatif dans le troisième volume. L'événement en question peut être le fait que la personne tente de sortir ou sorte de son lit ou qu'elle soit tombée de son lit. En revanche, l'unité de traitement ne déterminera pas qu'un événement concernant la personne a lieu si un mouvement est d'abord détecté dans le troisième volume (3) et que la détection de ce mouvement est suivie dans le premier laps de temps de la détection d'un mouvement dans le deuxième volume (2). Ainsi le dispositif ne détectera rien s'il s'agit du mouvement d'une personne s'approchant du lit, ce qui est bien le but poursuivi dans cette version préférée.

Le premier laps de temps est par exemple un laps de temps de5 secondes, de préférence de 4 secondes, de préférence de 3 secondes, de préférence de 2 secondes, de préférence de 1 seconde, et de façon préférentielle de 0,5 seconde.

Dans chacun des cas précédents, l'unité de traitement (30) est de préférence configurée pour déterminer un événement concernant ladite personne si les distances mesurées pour au moins un nombre prédéterminé d'obstacles situés dans le deuxième volume (2) varient chacune de plus qu'une première valeur prédéterminée, ledit nombre prédéterminé d'obstacles étant supérieur ou égal à deux. L'événement en question peut être le fait que la personne tente de sortir ou sorte de son lit ou qu'elle soit tombée de son lit. Selon cette version préférée, on évite que le dispositif ne détecte un événement si seulement un faible volume matériel se déplace dans le deuxième volume, tel que par exemple si la personne alitée fait uniquement pendre son bras à l'extérieur du premier volume (1). On évite ainsi de fausses alertes.

De préférence, le premier volume (1), le deuxième volume (2) et le troisième volume (3) sont chacun formés d'un ou plusieurs parallélépipèdes, de préférence des parallélépipèdes rectangles comme illustré par exemple à la Fig.1.

De façon préférentielle, le premier volume (1) est limité latéralement par des surfaces perpendiculaires à la surface supérieure du lit et tangentes aux bords du lit (40), et par une surface supérieure horizontale et disposée à maximum 100 cm de la surface supérieure du lit, ou disposée à 80 cm de la surface supérieure du lit, voire disposée à 50 cm de la surface supérieure du lit. La surface supérieure du lit est par exemple la surface supérieure du matelas du lit lorsque le matelas est à l'horizontale et que le lit est dans une position la plus haute verticalement.

Alternativement, le premier volume (1), le deuxième volume (2) et le troisième volume (3) peuvent être formés de sphères concentriques et centrées sur le détecteur.

Dans le cas de parallélépipèdes rectangles, le deuxième volume (2) est de préférence formé par un ensemble de points de l'espace se situant chacun à une distance horizontale DH (voir Fig. 3) comprise entre zéro et deux mètres des limites latérales du premier volume (1). De manière plus préférée, le deuxième volume est formé par un ensemble de points de l'espace se situant chacun à une distance horizontale DH (voir Fig. 3) comprise entre zéro et un mètre des limites latérales du premier volume. De manière encore plus préférée, le deuxième volume est formé par un ensemble de points de l'espace se situant chacun à une distance horizontale DH (voir Fig. 3) comprise entre zéro et un 50 cm des limites latérales du premier volume. De préférence, le deuxième volume est un volume s'étendant à partir du sol jusqu'au moins une hauteur d'installation du détecteur (20).

Dans chacun des cas précédents ou alternativement, l'unité de traitement (30) est de préférence configurée pour calculer la position du centre géométrique d'un groupe d'obstacles situés dans le deuxième volume (2) et dont les distances mesurées varient chacune de plus que la première valeur prédéterminée, et pour déterminer que la personne a chutée lorsque ledit centre géométrique se situe à une hauteur inférieure à une hauteur de référence. Une telle version préférée permet en effet de détecter que la personne a fait une chute. Il est à noter qu'il ne faut pas confondre ici le centre géométrique du groupe d'obstacles, qui est la position moyenne des points du groupe d'obstacles dans l'espace, de son centre de gravité ou de son centre de masse.

De préférence, la hauteur de référence est la hauteur de la surface supérieure du lit par rapport au sol sur lequel il repose. Il s'agit par exemple de la hauteur de la surface supérieure du matelas du lit.

De préférence, l'unité de traitement (30) comporte une mémoire dans laquelle la première valeur prédéterminée est stockée et/ou dans laquelle la deuxième valeur prédéterminée est stockée et/ou dans laquelle la hauteur de référence est stockée.

La première valeur prédéterminée peut être stockée dans cette mémoire lors de la fabrication du dispositif (10), ou enregistrée suite à des mesures empiriques lors de la mise en place du dispositif, en réalisant des tests avec la personne alitée. Dans ce dernier cas, la première valeur déterminée est choisie lors de l'application du dispositif au sein de la chambre d'une personne et est stockée à ce moment dans la mémoire. Il s'agit d'une valeur qui est par exemple considérée comme signifiant qu'une variation de distance correspondant à un mouvement non négligeable a été détectée par le dispositif dans le deuxième volume (2). Il en va de même pour la deuxième valeur prédéterminée.

La première valeur prédéterminée peut contenir une pluralité de valeurs correspondant à une pluralité de morphologies, afin d'adapter le dispositif à des personnes de taille variable. La première valeur prédéterminée peut correspondre à une variation de distance minimum, par exemple une variation de distance supérieure à 5 cm, voire supérieure à 10 cm, voire supérieure à 15 cm, et préférentiellement supérieure à 20 cm. Alternativement ou complémentairement, la supérieure valeur prédéterminée peut correspondre à un nombre minium de capteurs pour lesquels une variation de distance a été mesurée. Cette valeur peut aussi correspondre à un pourcentage minimum ou une proportion minimum de capteurs de distance associés à un volume particulier pour lequel une variation de distance a été mesurée, comme par exemple plus de 5% des capteurs de distance, ou préférentiellement plus de 10% des capteurs de distance, ou plus préférentiellement plus de 20% des capteurs de distance. Complémentairement, la supérieure valeur prédéterminée peut correspondre à la mesure d'une variation de distance pour un nombre ou un pourcentage minimum de capteurs de distance présentant une variation de distance supérieure à une distance minimum. Ainsi, et à titre d'exemple, une supérieure valeur prédéterminée peut correspondre à au moins 1 capteur de distance, ou à au moins 5 capteurs de distance ou à au moins 10 capteurs de distance, ou 10% de capteurs de distance associés à une volume dont la variation de distance est supérieure à 5 cm, ou à 10 cm. D'autres combinaisons sont possibles. La première valeur prédéterminée peut comprendre une pluralité de valeurs, chacune associée à une valeur seuil associée à chaque volume (1, 2). En effet, les mouvements peuvent avoir des amplitudes différentes dans les différents volumes, et avoir une pluralité de valeurs seuil, chacune correspondant à un volume particulier, ce qui permet d'avoir un traitement plus fin de l'information.

Il en va de même pour la deuxième valeur prédéterminée.

De préférence, le dispositif (10) comprend des moyens visuels et/ou sonores et l'unité de traitement (30) est configurée pour activer lesdits moyens visuels et/ou sonores lorsque ledit événement concernant ladite personne est détecté. Ceci permet à une tierce personne de se rendre compte que ledit événement concernant ladite personne est détecté. Ces moyens visuels et/ou sonores sont de préférence placés à distance du détecteur (10), par exemple dans une autre pièce que celle où se trouve le lit surveillé (40), tel qu'un poste de surveillance d'un centre de soin par exemple.

Alternativement, le dispositif (10) peut comprendre des moyens pour transmettre un signal d'alerte à un objet tiers lorsqu'un événement concernant la personne est détecté, l'objet tiers pouvant alors activer des moyens visuels et/ou sonores lorsqu'il reçoit un tel signal d'alerte et/ou transmettre ce signal d'alerte à encore un autre objet tiers. L'objet tiers peur par exemple être un smartphone sur lequel tourne une application rendant le smartphone apte à recevoir ledit signal d'alerte et à faire retentir une alarme sonore et/ou afficher une information indiquant l'occurrence de l'événement en question. Alternativement, le signal d'alerte peut être un signal transitoire stocké en mémoire, ne déclenchant aucune alarme, et apte à déclencher une alarme si certaines conditions supplémentaires sont réunies.

Dans une version préférentielle du dispositif (10), l'unité de traitement (30) est configurée pour stocker le signal d'alerte en mémoire et pour déclencher une alarme ou envoyer un signal d'alerte en fonction de l'analyse des signaux d'alerte subséquents. Ainsi, lorsque l'unité de traitement a émis un signal d'alerte suite à l'analyse des variations de distance au sein des trois volumes (1, 2, 3), l'unité de traitement est configurée pour supprimer ce signal d'alerte si aucune variation subséquente de distance n'est mesurée successivement au sein du troisième volume, au sein du second volume, et enfin au sein du premier volume. L'unité de traitement (30) est de préférence configurée pour limiter cette possibilité de suppression du signal d'alerte durant une période de temps limitée. Ainsi, si après une période de temps supérieure à 1 minute, voire supérieure à 2 minutes, voire supérieure à 3 minutes, voire supérieure à 4 minutes, ou encore supérieure à 5 minutes, aucune variation de distance n'est mesurée successivement du troisième volume au premier volume, l'unité de traitement (30) est configurée pour émettre le signal d'alerte afin d'activer un quelconque moyen visuel ou sonore tel que décrit précédemment.

Selon ce dispositif préférentiel, aucune alarme n'est généré lorsque des mouvements sont détectés depuis l'extérieur de l'espace surveillé jusqu'au lit, signifiant soit que la personne précédemment alitée s'est recouchée, soit qu'une tierce personne est intervenue, et a accompagné le patient vers son lit, réduisant ainsi le déclenchement d'une alerte non désirée.

La présente invention concerne également une méthode pour détecter si une personne alitée quitte son lit (40), la méthode comprenant les étapes suivantes :
a) mise en place d'un détecteur (20) de manière à ce qu'un champ de détection (50) du détecteur couvre au moins une partie du lit (40) et au moins une partie de son environnement, ledit détecteur (20) comportant plusieurs capteurs de distance, chaque capteur de distance étant apte à fournir des mesures de distance au cours du temps entre ledit capteur et un obstacle correspondant dans ledit champ de détection (50), ledit détecteur (20) étant relié à une unité de traitement (30) configurée pour traiter les mesures de distance fournies par les capteurs de distance du détecteur,
b) calibration du détecteur et de l'unité de traitement pour définir un espace à surveiller dans le champ de détection du détecteur, cet espace à surveiller étant divisé en au moins un premier volume (1) et un deuxième volume (2), le premier volume étant un volume s'étendant verticalement vers le haut et/ou vers le bas à partir de la surface dudit lit, le deuxième volume étant un volume s'étendant vers l'extérieur à partir des limites latérales du premier volume,
c) déterminer, au moyen de l'unité de traitement (30), la position dans l'espace de chacun desdits obstacles au cours du temps et en fonction de la distance mesurée par le détecteur pour l'obstacle en question,
d) classer, au moyen de l'unité de traitement (30), chaque obstacle selon que l'obstacle est situé dans le premier volume ou dans le deuxième volume,
e) déterminer, au moyen de l'unité de traitement (30), qu'un événement concernant ladite personne a lieu si la distance mesurée pour au moins un obstacle situé dans le deuxième volume varie de plus qu'une première valeur prédéterminée.

La présente invention a été décrite en relation avec des modes de réalisation spécifiques, qui ont une valeur purement illustrative et ne doivent pas être considérés comme limitatifs. D'une manière générale, il apparaîtra évident pour l'homme du métier que la présente invention n'est pas limités aux exemples illustrés et/ou décrits ci-dessus. La présence de numéros de référence aux dessins ne peut être considérée comme limitative, y compris lorsque ces numéros sont indiqués dans les revendications.

L'usage des verbes « comprendre », « inclure », « comporter », ou toute autre variante, ainsi que leurs conjugaisons, ne peut en aucune façon exclure la présence d'capteurs autres que ceux mentionnés.

L'usage de l'article indéfini « un », « une », ou de l'article défini « le », « la » ou « l' », pour introduire un capteur n'exclut pas la présence d'une pluralité de ces capteurs.

L'invention peut également être décrite comme suit : un dispositif (10) et une méthode pour détecter si une personne alitée tente de quitter son lit ou quitte son lit ou si elle a chuté. Le dispositif effectue une analyse au cours du temps des distances entre les capteurs d'un détecteur (20) et les objets présents dans le champ de détection (50) du détecteur. La détection se base sur des variations desdites distances, et de leurs localisations au sein de l'espace (60) surveillé.

## Revendications

1. Dispositif (10) pour détecter si une personne alitée quitte son lit ou a quitté son lit ou a chuté après avoir quitté son lit, le dispositif (10) comprenant un détecteur (20) ayant un champ de détection (50) apte à couvrir au moins une partie du lit (40) et au moins une partie de son environnement, ledit détecteur (20) comportant plusieurs capteurs de distance, chaque capteur de distance étant apte à fournir des mesures de distance au cours du temps entre ledit capteur et un obstacle correspondant dans ledit champ de détection (50), ledit dispositif (10) comprenant en outre une unité de traitement (30) reliée au détecteur (20) et configurée pour traiter les mesures de distance fournies par les capteurs de distance du détecteur,
**caractérisé en ce que** l'unité de traitement (30) est configurée pour déterminer la position dans l'espace de chacun desdits obstacles au cours du temps et en fonction de la distance mesurée par le détecteur pour l'obstacle en question, et pour classer chaque obstacle selon que l'obstacle est situé dans un premier volume (1) ou dans un deuxième volume (2) dans l'espace, le premier volume (1) étant un volume s'étendant verticalement vers le haut et/ou vers le bas à partir de la surface dudit lit, le deuxième volume (2) étant un volume s'étendant vers l'extérieur à partir des limites latérales du premier volume,
et **en ce que** l'unité de traitement (30) est configurée pour déterminer un événement concernant ladite personne si la distance mesurée pour au moins un obstacle situé dans le deuxième volume varie au cours du temps de plus qu'une première valeur prédéterminée.

2. Dispositif (10) selon la revendication 1, **caractérisé en ce que** l'unité de traitement (30) est configurée pour classer chaque obstacle selon que l'obstacle est situé dans le premier volume (1) ou dans le deuxième volume (2) ou dans un troisième volume (3), le troisième volume étant un volume s'étendant vers l'extérieur à partir des limites latérales extérieures du deuxième volume,
et **en ce que** l'unité de traitement (30) est configurée pour déterminer un événement concernant ladite personne si la distance mesurée pour au moins un obstacle situé dans le deuxième volume (2) varie de plus qu'une première valeur prédéterminée, à condition qu'aucune des distances mesurées pour les obstacles situé dans le troisième volume (3) n'aient varié de plus qu'une deuxième valeur prédéterminée pendant un premier laps de temps précédent le moment où la distance mesurée pour l'au moins un obstacle situé dans le deuxième volume a varié de plus que la première valeur prédéterminée.

3. Dispositif (10) selon la revendication 1 ou 2, **caractérisé en ce que** l'unité de traitement (30) est configurée pour déterminer un événement concernant ladite personne si les distances mesurées pour au moins un nombre prédéterminé d'obstacles situés dans le deuxième volume (2) varient chacune de plus qu'une première valeur prédéterminée, ledit nombre prédéterminé d'obstacles étant supérieur ou égal à deux.

4. Dispositif (10) selon l'une quelconque des revendications précédentes, **caractérisé en ce que** le premier volume (1), le deuxième volume (2) et le troisième volume (3) sont chacun formés d'un ou plusieurs parallélépipèdes, de préférence des parallélépipèdes rectangles.

5. Dispositif (10) selon la revendication 4, **caractérisé en ce que** le deuxième volume (2) est formé par un ensemble de points de l'espace se situant chacun à une distance horizontale comprise entre zéro et deux mètres des limites latérales du premier volume (1).

6. Dispositif (10) selon la revendication 5, **caractérisé en ce que** le deuxième volume (2) est formé par un ensemble de points de l'espace se situant chacun à une distance horizontale comprise entre zéro et un mètre des limites latérales du premier volume (1).

7. Dispositif (10) selon l'un quelconque des revendications précédentes, **caractérisé en ce que** le détecteur (20) est placé ou configuré pour être placé à une hauteur supérieure à la hauteur maximale de la surface supérieure dudit lit (40).

8. Dispositif (10) selon l'un quelconque des revendications précédentes, **caractérisé en ce que** le deuxième volume (2) est un volume s'étendant à partir du sol jusqu'au moins une hauteur d'installation du détecteur (20).

9. Dispositif (10) selon l'un quelconque des revendications précédentes, **caractérisé en ce que** l'événement est une sortie du lit dans lequel la personne était alitée ou une chute de ladite personne.

10. Dispositif (10) selon l'un quelconque des revendications précédentes, **caractérisé en ce que** l'unité de traitement (30) est configurée pour calculer la position du centre géométrique d'un groupe d'obstacles situés dans le deuxième volume (2) et dont les distances mesurées varient chacune de plus que la première valeur prédéterminée, et pour déterminer que la personne a chutée lorsque ledit centre géométrique se situe à une hauteur inférieure à une hauteur de référence.

11. Dispositif (10) selon la revendication 10, **caractérisé en ce que** la hauteur de référence est la hauteur de la surface supérieure du lit (40) par rapport au sol sur lequel il repose.

12. Dispositif (10) selon l'une quelconque des revendications précédentes, **caractérisé en ce que** l'unité de traitement (30) comporte une mémoire dans laquelle la première valeur prédéterminée et/ou la deuxième valeur prédéterminée est stockée.

13. Dispositif (10) selon l'une quelconque des revendications précédentes, **caractérisé en ce que** le dispositif (10) comprend des moyens visuels et/ou sonores et **en ce que** l'unité de traitement (30) est configurée pour activer lesdits moyens visuels et/ou sonores lorsque ledit événement concernant ladite personne est détecté.

14. Méthode pour détecter si une personne alitée quitte son lit ou a quitté son lit ou a chuté après avoir quitté son lit, la méthode comprenant les étapes suivantes :
a) mise en place d'un détecteur (20) de manière à ce qu'un champ de détection (50) du détecteur couvre au moins une partie du lit (40) et au moins une partie de son environnement, ledit détecteur (20) comportant plusieurs capteurs de distance, chaque capteur de distance étant apte à fournir des mesures de distance au cours du temps entre ledit capteur et un obstacle correspondant dans ledit champ de détection (50), ledit détecteur (20) étant relié à une unité de traitement (30) configurée pour traiter les mesures de distance fournies par les capteurs de distance du détecteur,
b) calibration du détecteur et de l'unité de traitement pour définir un espace à surveiller dans le champ de détection du détecteur, cet espace à surveiller étant divisé en au moins un premier volume (1) et un deuxième volume (2), le premier volume étant un volume s'étendant verticalement vers le haut et/ou vers le bas à partir de la surface dudit lit, le deuxième volume étant un volume s'étendant vers l'extérieur à partir des limites latérales du premier volume,
c) déterminer, au moyen de l'unité de traitement (30), la position dans l'espace de chacun desdits obstacles au cours du temps et en fonction de la distance mesurée par le détecteur pour l'obstacle en question,
d) classer, au moyen de l'unité de traitement (30), chaque obstacle selon que l'obstacle est situé dans le premier volume (1) ou dans le deuxième volume (2),
e) déterminer, au moyen de l'unité de traitement (30), qu'un événement concernant ladite personne a lieu si la distance mesurée pour au moins un obstacle situé dans le deuxième volume (2) varie au cours du temps de plus qu'une première valeur prédéterminée.

## Patentansprüche

1. Vorrichtung (10) zum Erkennen, ob eine bettlägerige Person ihr Bett verlässt oder ihr Bett verlassen hat oder nach dem Verlassen ihres Bettes gestürzt ist, wobei die Vorrichtung (10) einen Detektor (20) mit einem Erfassungsfeld (50) aufweist, das zumindest einen Teil des Bettes (40) und zumindest einen Teil seiner Umgebung abdecken kann, wobei der Detektor (20) mehrere Abstandssensoren aufweist, wobei jeder Abstandssensor in der Lage ist, Abstandsmessungen über die Zeit zwischen dem Sensor und einem entsprechenden Hindernis in dem Erfassungsfeld (50) bereitzustellen, wobei die Vorrichtung (10) weiterhin eine Verarbeitungseinheit (30) umfasst, die mit dem Sensor (20) verbunden und konfiguriert ist um die von den Abstandssensoren des Sensors bereitgestellten Abstandsmessungen zu verarbeiten, **dadurch gekennzeichnet, dass** die Behandlungseinheit (30) so konfiguriert ist, dass sie die räumliche Position jedes der Hindernisse im Laufe der Zeit und entsprechend der vom Detektor für das betreffende Hindernis gemessenen Entfernung bestimmt und jedes Hindernis danach klassifiziert, ob sich das Hindernis in einem ersten Volumen (1) oder in einem zweiten Volumen (2) im Raum befindet, wobei das erste Volumen (1) ein Volumen ist, das sich vertikal nach oben und/oder unten von der Oberfläche des Bettes erstreckt, das zweite Volumen (2) ein Volumen ist, das sich von den seitlichen Grenzen des ersten Volumens nach außen erstreckt, und dass die Behandlungseinheit (30) so konfiguriert ist, dass sie ein Ereignis bestimmt, das diese Person betrifft, wenn der gemessene Abstand für mindestens ein Hindernis im zweiten Volumen im Laufe der Zeit um mehr als einen ersten vorgegebenen Wert variiert.

2. Vorrichtung (10) nach Anspruch 1, **dadurch gekennzeichnet, dass** die Behandlungseinheit (30) so konfiguriert ist, dass sie jedes Hindernis danach klassifiziert, ob sich das Hindernis im ersten Volumen (1) oder im zweiten Volumen (2) oder in einem dritten Volumen (3) befindet, wobei das dritte Volumen ein Volumen ist, das sich von den äußeren seitlichen Grenzen des zweiten Volumens nach außen erstreckt, und dass die Verarbeitungseinheit (30) so konfiguriert ist, dass sie ein Ereignis bestimmt, das diese Person betrifft, wenn die Entfernung, die für mindestens ein Hindernis im zweiten Volumen (2) gemessen wird, um mehr als einen ersten vorgegebenen Wert variiert, vorausgesetzt, dass keine der Entfernungen, die für die im dritten Volumen (3) befindlichen Hindernisse gemessen wurden, während eines ersten Zeitraums vor dem Zeitpunkt, zu dem die gemessene Entfernung für mindestens ein Hindernis, das sich im zweiten Volumen befindet, um mehr als den ersten vorgegebenen Wert variiert.

3. Vorrichtung (10) nach Anspruch 1 oder 2, **dadurch gekennzeichnet, dass** die Verarbeitungseinheit (30) so konfiguriert ist, dass sie ein Ereignis bestimmt, das diese Person betrifft, wenn die Entfernungen, die für mindestens eine vorbestimmte Anzahl von Hindernissen gemessen werden, die sich im zweiten Volumen (2) befinden, jeweils um mehr als einen ersten vorgegebenen Wert variieren, wobei die vorgegebene Anzahl von Hindernissen größer oder gleich zwei ist.

4. Vorrichtung (10) nach einem der vorhergehenden Ansprüche, **dadurch gekennzeichnet, dass** das erste Volumen (1), das zweite Volumen (2) und das dritte Volumen (3) jeweils aus einem oder mehreren Quaderepipeden, vorzugsweise rechteckigen Quaderepipeden, ausgebildet sind.

5. Vorrichtung (10) nach Anspruch 4, **dadurch gekennzeichnet, dass** das zweite Volumen (2) durch eine Reihe von Punkten im Raum gebildet wird, von denen jeder in einem horizontalen Abstand zwischen null und zwei Metern von den seitlichen Grenzen des ersten Volumens (1) liegt.

6. Vorrichtung (10) nach Anspruch 5, **dadurch gekennzeichnet, dass** das zweite Volumen (2) durch eine Reihe von Punkten im Raum gebildet wird, von denen jeder in einem horizontalen Abstand zwischen Null und einem Meter von den seitlichen Grenzen des ersten Volumens (1) liegt.

7. Vorrichtung (10) nach einem der vorhergehenden Ansprüche, **dadurch gekennzeichnet, dass** der Detektor (20) in einer Höhe angeordnet oder konfiguriert ist, die größer ist als die maximale Höhe der Oberseite des Bettes (40).

8. Vorrichtung (10) nach einem der vorhergehenden Ansprüche, **dadurch gekennzeichnet, dass** das zweite Volumen (2) ein Volumen ist, das sich vom Boden bis zu mindestens einer Einbauhöhe des Detektors (20) erstreckt.

9. Vorrichtung (10) nach einem der vorhergehenden Ansprüche, **dadurch gekennzeichnet, dass** das Ereignis ein Ausgang aus dem Bett, in dem die Person bettlägerig war, oder ein Sturz dieser Person ist.

10. Vorrichtung (10) nach einem der vorhergehenden Ansprüche, **dadurch gekennzeichnet, dass** die Verarbeitungseinheit (30) so konfiguriert ist, dass sie die Position des geometrischen Mittelpunkts einer Gruppe von Hindernissen berechnet, die sich im zweiten Volumen (2) befinden und deren gemessene Abstände jeweils mehr als der erste vorgegebene Wert variieren, und um zu bestimmen, dass die Person gestürzt ist, wenn sich der geometrische Mittelpunkt in einer Höhe unterhalb einer Referenzhöhe befindet.

11. Vorrichtung (10) nach Anspruch 10, **dadurch gekennzeichnet, dass** die Bezugshöhe die Höhe der Oberseite des Bettes (40) in Bezug auf den Boden ist, auf dem es aufliegt.

12. Vorrichtung (10) nach einem der vorhergehenden Ansprüche, **dadurch gekennzeichnet, dass** die Verarbeitungseinheit (30) einen Speicher umfasst, in dem der erste vorgegebene Wert und/oder der zweite vorgegebene Wert gespeichert ist.

13. Vorrichtung (10) nach einem der vorhergehenden Ansprüche, **dadurch gekennzeichnet, dass** die Vorrichtung (10) visuelle und/oder akustische Mittel umfasst und dass die Verarbeitungseinheit (30) so konfiguriert ist, dass sie die visuellen und/oder akustischen Mittel aktiviert, wenn das Ereignis in Bezug auf die Person erkannt wird.

14. Verfahren zum Erkennen, ob eine bettlägerige Person ihr Bett verlässt oder ihr Bett verlassen hat oder nach dem Verlassen ihres Bettes gestürzt ist, wobei das Verfahren die folgenden Schritte umfasst:
a) Aufstellen eines Detektors (20) in der Weise, dass ein Erfassungsfeld (50) des Detektors mindestens einen Teil des Bettes (40) und zumindest einen Teil seiner Umgebung abdeckt, wobei der Sensor (20) mehrere Abstandssensoren umfasst, wobei jeder Abstandssensor in der Lage ist, Abstandsmessungen über die Zeit zwischen dem Sensor und einem entsprechenden Hindernis in dem Erfassungsfeld (50) zu liefern, wobei der Detektor (20) mit einer Verarbeitungseinheit (30) verbunden ist, die so konfiguriert ist, dass sie die von den Abstandssensoren des Detektors gelieferten Entfernungsmessungen verarbeitet,
b) Kalibrierung des Detektors und der Behandlungseinheit zur Definition eines zu überwachenden Raums im Erfassungsfeld des Detektors, wobei der zu überwachende Raum in mindestens ein erstes Volumen (1) und ein zweites Volumen (2) unterteilt ist, wobei das erste Volumen ein Volumen ist, das sich vertikal nach oben und/oder unten von der Oberfläche des Bettes erstreckt, das zweite Volumen ist ein Volumen, das sich von den seitlichen Grenzen des ersten Volumens nach außen erstreckt,
c) bestimmen mit Hilfe der Behandlungseinheit (30) die räumliche Lage jedes dieser Hindernisse im Zeitverlauf und in Abhängigkeit von der Entfernung, die der Detektor für das betreffende Hindernis gemessen hat;
d) jedes Hindernis mit Hilfe der Behandlungseinheit (30) danach zu klassifizieren, ob sich das Hindernis im ersten Volumen (1) oder im zweiten Volumen (2) befindet;
e) mit Hilfe der Verarbeitungseinheit (30) feststellen, dass ein Ereignis eintritt, das diese Person betrifft, wenn der gemessene Abstand für mindestens ein Hindernis im zweiten Volumen im Laufe der Zeit um mehr als einen ersten vorgegebenen Wert variiert.

## Claims

1. Device (10) for detecting whether a bedridden person leaves his bed or has left his bed or has fallen after leaving his bed, the device (10) comprising a detector (20) having a detection field (50) capable of covering at least part of the bed (40) and at least part of its environment, said detector (20) having several distance sensors, wherein each distance sensor is capable of providing distance measurements over time between said sensor and a corresponding obstacle in said detection field (50), said device (10) further comprising a processing unit (30) connected to the sensor (20) and configured to process the distance measurements provided by the distance sensors of the sensor, **characterised in that** the treatment unit (30) is configured to determine the spatial position of each of said obstacles over time and according to the distance measured by the detector for the obstacle in question, and to classify each obstacle according to whether the obstacle is located in a first volume (1) or in a second volume (2) in space, the first volume (1) being a volume extending vertically upwards and/or downwards from the surface of said bed, the second volume (2) being a volume extending outwards from the lateral limits of the first volume,
and **in that** the treatment unit (30) is configured to determine an event concerning said person if the distance measured for at least one obstacle located in the second volume varies over time by more than a first predetermined value.

2. Device (10) according to claim 1, **characterized in that** the treatment unit (30) is configured to classify each obstacle according to whether the obstacle is located in the first volume (1) or in the second volume (2) or in a third volume (3), the third volume being a volume extending outwards from the outer lateral limits of the second volume, and **in that** the processing unit (30) is configured to determine an event concerning said person if the distance measured for at least one obstacle located in the second volume (2) varies by more than a first predetermined value, provided that none of the distances measured for the obstacles located in the third volume (3) have varied by more than a second predetermined value during a first period of time preceding the time when the measured distance for at least one obstacle located in the second volume varied by more than the first predetermined value.

3. Device (10) according to claim 1 or 2, **characterized in that** the processing unit (30) is configured to determine an event concerning said person if the distances measured for at least a predetermined number of obstacles located in the second volume (2) each vary by more than a first predetermined value, said predetermined number of obstacles being greater than or equal to two.

4. Device (10) according to any one of the preceding claims, **characterized in that** the first volume (1), the second volume (2) and the third volume (3) are each formed of one or more parallelepipeds, preferably rectangular parallelepipeds.

5. Device (10) according to claim 4, **characterized in that** the second volume (2) is formed by a set of points in space, each of which lies at a horizontal distance between zero and two meters from the lateral limits of the first volume (1).

6. Device (10) according to claim 5, **characterized in that** the second volume (2) is formed by a set of points in space, each of which is at a horizontal distance between zero and one meter from the lateral limits of the first volume (1).

7. A device (10) according to any one of the preceding claims, **characterized in that** the detector (20) is placed or configured to be placed at a height greater than the maximum height of the upper surface of said bed (40).

8. A device (10) according to any one of the preceding claims, **characterized in that** the second volume (2) is a volume extending from the ground to at least one installation height of the detector (20).

9. A device (10) according to any one of the preceding claims, **characterized in that** the event is an exit from the bed in which the person was bedridden or a fall of said person.

10. Device (10) according to any one of the preceding claims, **characterized in that** the processing unit (30) is configured to calculate the position of the geometric center of a group of obstacles located in the second volume (2) and whose measured distances each vary more than the first predetermined value, and to determine that the person has fallen when said geometric center is at a height below a reference height.

11. Device (10) according to claim 10, **characterized in that** the reference height is the height of the upper surface of the bed (40) in relation to the ground on which it rests.

12. A device (10) according to any one of the preceding claims,
**characterized in that** the processing unit (30) comprises a memory in which the first predetermined value and/or the second predetermined value is stored.

13. A device (10) according to any one of the preceding claims,
**characterized in that** the device (10) comprises visual and/or audible means and **in that** the processing unit (30) is configured to activate said visual and/or audible means when said event concerning said person is detected.

14. A method for detecting whether a bedridden person is leaving their bed or has left their bed or has fallen after leaving their bed, the method comprising the following steps:
a) setting up a detector (20) such that a detection field (50) of the detector covers at least part of the bed (40) and at least part of its surroundings, said sensor (20) comprising several distance sensors, each distance sensor being capable of providing distance measurements over time between said sensor and a corresponding obstacle in said detection field (50), said detector (20) being connected to a processing unit (30) configured to process the distance measurements provided by the distance sensors of the detector,
b) calibration of the detector and the treatment unit to define a space to be monitored in the detection field of the detector, said space to be monitored being divided into at least a first volume (1) and a second volume (2), the first volume being a volume extending vertically upwards and/or downwards from the surface of said bed, the second volume being a volume extending outwards from the lateral limits of the first volume,
c) determine, by means of the treatment unit (30), the position in space of each of those obstacles over time and as a function of the distance measured by the detector for the obstacle in question,
d) classify, by means of the treatment unit (30), each obstacle according to whether the obstacle is located in the first volume (1) or in the second volume (2),
e) determine, by means of the processing unit (30), that an event concerning that person takes place if the distance measured for at least one obstacle located in the second volume (2) varies over time by more than a first predetermined value.
